# EUROPEAN PATENT SPECIFICATION

(11) **EP 3 603 674 B1**
(45) Date of publication and mention of the grant of the patent: **26.02.2025**
(21) Application number: 19194893.4
(22) Date of filing: 30.10.2009
(51) Int. Cl.: A61K 45/06, A61K 31/485, A61P 25/00, A61K 31/15, A61K 31/135, A61K 31/138, A61K 31/343, A61K 31/4525, A61P 25/04

(54) **COMBINATION OF MORPHINAN COMPOUNDS AND ANTIDEPRESSANT FOR THE TREATMENT OF INTRACTABLE AND CHRONIC PAIN**
KOMBINATION VON MORPHINANVERBINDUNGEN UND ANTIDEPRESSIVUM ZUR BEHANDLUNG VON HARTNÄCKIGEN UND CHRONISCHEN SCHMERZEN
COMBINAISON DE COMPOSÉS DE MORPHINE ET ANTIDÉPRESSEUR POUR LE TRAITEMENT DES DOULEURS RÉFRACTAIRES ET CHRONIQUES

(30) Priority: 30.10.2008 US 109832 P
(43) Date of publication of application: 05.02.2020
(62) Divisional of application: 16166277.0
(73) Proprietor: Sun Pharmaceutical Industries, Inc., Princeton, NJ 08540 (US)
(72) Inventor: THOMAS, Amanda, Belmont, MA Massachusetts 02478 (US)
(74) Representative: Marks & Clerk LLP

(56) References cited:
- WO-A1-2008/137474
- WO-A1-96/09044
- WO-A2-2008/097924
- EICHHOLD T H ET AL: "Highly sensitive high-performance liquid chromatographic-tandem mass spectrometric method for the analysis of dextromethorphan in human plasma", JOURNAL OF CHROMATOGRAPHY B: BIOMEDICAL SCIENCES & APPLICATIONS, ELSEVIER, AMSTERDAM, NL, vol. 698, no. 1-2, 26 September 1997 (1997-09-26), pages 147 - 154, XP004092128, ISSN: 1570-0232, DOI: 10.1016/S0378-4347(97)00308-3
- HEINKELE G ET AL: "Synthesis of [2H3]-dextromethorphan and its major urinary metabolites [2H3]-dextrorphan and [2H3]-dextrorphan-beta-glucoronide", JOURNAL OF LABELLED COMPOUNDS AND RADIOPHARMACEUTICALS, vol. 45, 1 January 2002 (2002-01-01), pages 1153 - 1158, XP002490642, ISSN: 0362-4803
- RICHTER G ET AL: "Synthesis of deuterated dextromethorphan derivatives", ARKIVOC : FREE ONLINE JOURNAL OF ORGANIC CHEMISTRY, vol. 2008, no. 3, 1 March 2008 (2008-03-01), pages 182 - 193, XP002490643, ISSN: 1551-7012
- IN CHE S ET AL: "An update on the pharmacological management of post-herpetic neuralgia and painful diabetic neuropathy", CNS DRUGS, ADIS INTERNATIONAL, AUCKLAND, NZ, vol. 22, no. 5, 1 January 2008 (2008-01-01), pages 417 - 442, XP009128602, ISSN: 1172-7047
- FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1 January 1985 (1985-01-01), pages 1 - 40, XP009086953, ISSN: 0065-2490
- SHAO ET AL: "The kinetic isotope effect in the search for deuterated drugs", DRUG NEWS AND PERSPECTIVES, PROUS SCIENCE, ES, vol. 23, no. 6, 1 January 2010 (2010-01-01), pages 398 - 404, XP009139025, ISSN: 0214-0934
- TUNG R: "The Development of Deuterium-Containing Drugs", INNOVATIONS IN PHARMACEUTICAL TECHNOLOGY, SAMEDAN LTD, GB, no. 32, 1 March 2010 (2010-03-01), pages 24 - 26, 28, XP009148260, ISSN: 1471-7204

## Description

### TECHNICAL FIELD

This invention relates to combinations for use in the treatment of chronic or intractable pain.

### BACKGROUND

Many current medicines suffer from poor absorption, distribution, metabolism and/or excretion (ADME) properties that prevent their wider use. Poor ADME properties are also a major reason for the failure of drug candidates in clinical trials. While formulation technologies and prodrug strategies can be employed in some cases to improve certain ADME properties, these approaches have failed to overcome the inherent ADME problems that exist for many drugs and drug candidates. One inherent problem is the rapid metabolism that causes a number of drugs, which otherwise would be highly effective in treating a disease, to be cleared too rapidly from the body. A possible solution to rapid drug clearance is frequent or high dosing to attain a sufficiently high plasma level of drug. This, however, introduces a number of potential treatment problems, such as poor patient compliance with the dosing regimen, side effects that become more acute with higher doses, and increased cost of treatment.

In some select cases, a metabolic inhibitor will be co-administered with an important drug that is rapidly cleared. Such is the case with the protease inhibitor class of drugs that are used to treat HIV infection. These drugs are typically co-dosed with ritonavir, an inhibitor of cytochrome P450 enzyme CYP3A4, the enzyme responsible for their metabolism. Ritonavir itself has side effects and it adds to the pill burden for HIV patients who must already take a combination of different drugs. Similarly, dextromethorphan which undergoes rapid CYP2D6 metabolism is being tested in combination with the CYP2D6 inhibitor quinidine for the treatment of pseudobulbar disease.

In general, combining drugs with cytochrome P450 inhibitors is not a satisfactory strategy for decreasing drug clearance. The inhibition of a CYP enzyme activity can affect the metabolism and clearance of other drugs metabolized by that same enzyme. This can cause those other drugs to accumulate in the body to toxic levels.

A potentially attractive strategy, if it works, for improving a drug's metabolic properties is deuterium modification. In this approach, one attempts to slow the CYP-mediated metabolism of a drug by replacing one or more hydrogen atoms with deuterium atoms. Deuterium is a safe, stable, non-radioactive isotope of hydrogen. Deuterium forms stronger bonds with carbon than hydrogen does. In select cases, the increased bond strength imparted by deuterium can positively impact the ADME properties of a drug, creating the potential for improved drug efficacy, safety, and tolerability. At the same time, because the size and shape of deuterium are essentially identical to hydrogen, replacement of hydrogen by deuterium would not be expected to affect the biochemical potency and selectivity of the drug as compared to the original chemical entity that contains only hydrogen.

Over the past 35 years, the effects of deuterium substitution on the rate of metabolism have been reported for a very small percentage of approved drugs (see, e.g., Blake, MI et al, J Pharm Sci, 1975, 64:367-91; Foster, AB, Adv Drug Res 1985, 14:1-40 ("Foster"); Kushner, DJ et al, Can J Physiol Pharmacol 1999, 79-88; Fisher, MB et al, Curr Opin Drug Discov Devel, 2006, 9:101-09 ("Fisher")). The results have been variable and unpredictable. For some compounds deuteration caused decreased metabolic clearance in vivo. For others, there was no change in metabolism. Still others demonstrated decreased metabolic clearance. The variability in deuterium effects has also led experts to question or dismiss deuterium modification as a viable drug design strategy for inhibiting adverse metabolism. (See Foster at p. 35 and Fisher at p. 101).

The effects of deuterium modification on a drug's metabolic properties are not predictable even when deuterium atoms are incorporated at known sites of metabolism. Only by actually preparing and testing a deuterated drug can one determine if and how the rate of metabolism will differ from that of its undeuterated counterpart. Many drugs have multiple sites where metabolism is possible. The site(s) where deuterium substitution is required and the extent of deuteration necessary to see an effect on metabolism, if any, will be different for each drug.

Dextromethorphan, also known by its chemical name (+)-3-methoxy-17-methyl-(9α, 13α, 14α)-morphinan, is currently one of the most widely used antitussives.

In addition to the physiological activity noted above, dextromethorphan is also an agonist of the σ2 receptor, an N-methyl-D-aspartate (NMDA) antagonist, and an α3β4 nicotinic receptor antagonist. Dextromethorphan inhibits neurotransmitters, such as glutamate, from activating receptors in the brain. Uptake of dopamine and serotonin are also inhibited.

Dextromethorphan is approved for use in over the counter cough suppressant products. It is currently in Phase I clinical trials for treating subjects with voice spasms, and Phase III clinical studies for treating Rett Syndrome (http://www. clinicaltrials.gov). Dextromethorphan is being studied with other drugs in a Phase II clinical trial characterizing pain processing mechanisms in subjects with irritable bowel syndrome (http://www.clinicaltrials.gov/). Dextromethorphan is also in Phase I clinical trials for treating hyperalgesia in methadone-maintained subjects (http://www.clinicaltrials.gov/).

Dextromethorphan when administered alone has also shown limited efficacy in the treatment of other diseases and conditions, including involuntary emotional expression disorder ("IEED") or pseudobulbar affect ("PBA"), neurodegenerative diseases, neuropathic pain, and brain injuries.

Although dextromethorphan has shown therapeutic effect in the above mentioned conditions and disorders, its rapid first-pass metabolism remains a major obstacle in the development of effective treatments. Dextromethorphan is metabolized in the liver. Degradation begins with O- and N-demethylation to form primary metabolites dextrorphan and 3-methoxy-morphinan, both of which are further N- and O- demethylated respectively to 3-hydroxy-morphinan. These three metabolites are believed to be therapeutically active. A major metabolic catalyst is the cytochrome P450 enzyme 2D6 (CYP2D6), which is responsible for the O-demethylation reactions of dextromethorphan and 3-methoxymorphinan. N-demethylation of dextromethorphan and dextrorphan are catalyzed by enzymes in the related CYP3A family. Conjugates of dextrorphan and 3-hydroxymorphinan can be detected in human plasma and urine within hours of its ingestion. Aspects of the metabolism discussed above are reflected in, for example, Takashima, T., et al., Drug Metab. Pharmacokinet., 2005, 20(3): 177-182.

A combination of dextromethorphan hydrobromide and quinidine sulfate is currently in Phase III clinical trials for the treatment of PBA in patients suffering from Alzheimer's disease, stroke, Parkinson's disease and traumatic brain injury. (http://www.clinicaltrials.gov). The co-administration of quinidine (a potent inhibitor of the cytochrome P450 enzyme 2D6) increases both the blood level and the duration of action of dextromethorphan.

PBA is a neurological disorder characterized by inappropriate and uncontrollable outbursts of crying, laughing, or other emotional displays, often times with no relevant trigger or disproportionate with the actual mood of the person. Although rarely life threatening, PBA can significantly impact a person's professional and social life. PBA is commonly associated with certain neurological disorders such as traumatic brain injury, multiple sclerosis, amyotrophic lateral sclerosis, Alzheimer's disease, Parkinson disease, traumatic brain injury, progressive supranuclear palsy, multiple systems atrophy, normal pressure hydrocephalus, olivopontine cerebellar atrophy, brain tumors, Wilson's disease, and stroke. Over one million people in the United States suffer from PBA.

At the present time there are no treatments specifically approved by the Food and Drug Administration for the treatment of PBA. First line treatment for PBA is limited to the off-label use of antidepressants. Studies have demonstrated the therapeutic effect of tricylic antidepressants and selective serotonin reuptake inhibitors in the treatment of PBA. These agents are believed to have PBA-specific therapeutic effects independent of their antidepressant action. Antidepressants that have shown a therapeutic effect include amitriptyline, nortriptyline, citalopram, fluoxetine, paroxetine, and sertraline.

Although treatments for neurological diseases and conditions are known, there still exists a need to develop more efficacious treatments. The present disclosure fulfills this need and has other related advantages.

Other publications include WO2008/097924 which discloses pharmaceutical compositions comprising certain dextromethorphan analogs and methods for treating neurological disorders, and WO96/09044 which discloses compositions comprising dextromethorphan and an inhibitor of debrisoquin hydrolase, including quinidine, useful in the preparation of medicaments for treatment of chronic or intractable conditions including intractable coughing, dermatitis, chronic pain and tinnitus.

### SUMMARY

In one embodiment, provided is a combination of:
(a) a therapeutically effective amount of a selective serotonin reuptake inhibitor; and
(b) a therapeutically effective amount of a compound of Formula **II**: 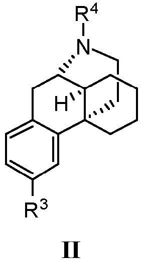 or a pharmaceutically acceptable salt thereof, wherein:
   R³ is selected from -OCH₂D, -OCHD₂, and -OCD₃;
   R⁴ is selected from -CH₃, -CH₂D, -CHD₂, and -CD₃;
   provided that when R³ is -OCD₃, then R⁴ is not -CH₃;
   for use in treating chronic or intractable pain.

In certain embodiments R⁴ is -CH₃, -CHD₂, or -CD₃. In certain embodiments R³ is - OCF₃, -OCD₃, or -OCHF₂.

In certain instances, the selective serotonin reuptake inhibitor is an inhibitor of a cytochrome p450 2D6 enzyme.

In certain instances, the selective serotonin reuptake inhibitor is selected from the group consisting of fluoxetine, norfluoxetine, citalopram, dapoxetine, escitalopram, fluvoxamine, paroxetine, and sertraline, or pharmaceutically acceptable salts thereof.

In certain instances, the selective serotonin reuptake inhibitor is selected from the group consisting of citalopram, norfluoxetine, dapoxetine, escitalopram, fluvoxamine, paroxetine, and sertraline, or pharmaceutically acceptable salts thereof.

In certain instances, the selective serotonin reuptake inhibitor is paroxetine, or a pharmaceutically acceptable salt thereof.

In certain instances, the selective serotonin reuptake inhibitor is selected from the group consisting of citalopram, fluvoxamine, norfluoxetine, fluoxetine, paroxetine and sertraline, and the compound of formula II is selected from the group consisting of: 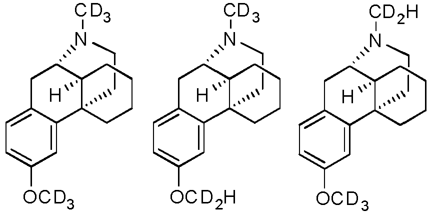 or pharmaceutically acceptable salts thereof; in certain instances, the selective serotonin reuptake inhibitor being selected from the group consisting of citalopram, fluvoxamine, paroxetine and sertraline, or a pharmaceutically acceptable salt thereof; in certain instances, the selective serotonin reuptake inhibitor being paroxetine, or a pharmaceutically acceptable salt thereof.

### BRIEF DESCRIPTION OF THE DRAWINGS

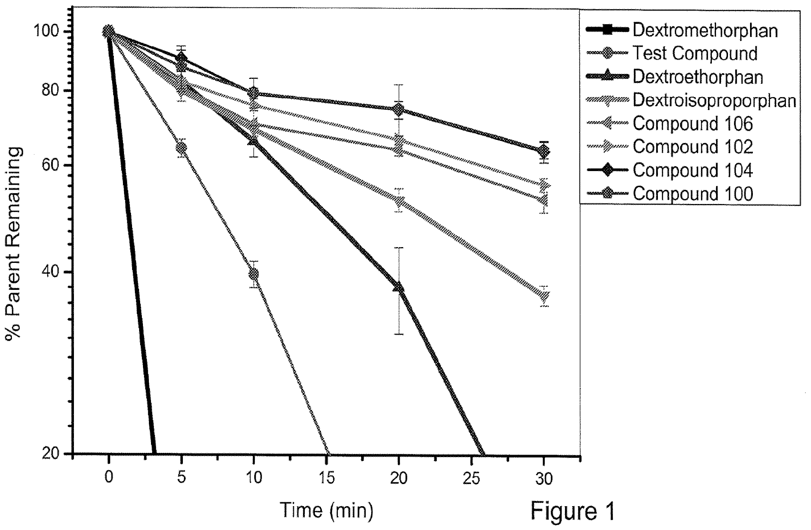

Figure 1 depicts the metabolic stability of compounds provided herein in CYP2D6 SUPERSOMES^{™}.

### DETAILED DESCRIPTION

### Definitions

The terms "ameliorate" and "treat" are used interchangeably and include both therapeutic treatment and/or prophylactic treatment (reducing the likelihood of development). Both terms mean decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

The term "alkyl" refers to a monovalent saturated hydrocarbon group. C₁-C₄ alkyl is an alkyl having from 1 to 4 carbon atoms. An alkyl may be linear or branched. Examples of alkyl groups include methyl; ethyl; propyl, including *n*-propyl and isopropyl; butyl, including *n*-butyl, isobutyl, *sec*-butyl, and *t*-butyl.

It will be recognized that some variation of natural isotopic abundance occurs in a synthesized compound depending upon the origin of chemical materials used in the synthesis. Thus, a preparation of dextromethorphan or dextromethorphan analogs will inherently contain small amounts of deuterated isotopologues. The concentration of naturally abundant stable hydrogen and carbon isotopes, notwithstanding this variation, is small and immaterial as compared to the degree of stable isotopic substitution of compounds provided herein. See, for instance, Wada E et al., Seikagaku 1994, 66:15; Gannes LZ et al., Comp Biochem Physiol Mol Integr Physiol 1998, 119:725.

Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 3340 times greater than the natural abundance of deuterium, which is 0.015% (i.e., the term "D" or "deuterium" indicates at least 50.1% incorporation of deuterium).

The term "isotopic enrichment factor" as used herein means the ratio between the isotopic abundance of D at a specified position in a compound provided herein and the naturally occurring abundance of that isotope.

In other embodiments, a compound provided herein has an isotopic enrichment factor for each deuterium present at a site designated as a potential site of deuteration on the compound of at least 3500 (52.5% deuterium incorporation), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term "isotopologue" refers to a species that has the same chemical structure and formula as a specific compound provided herein, with the exception of the positions of isotopic substitution and/or level of isotopic enrichment at one or more positions, e.g., H vs. D.

The term "compound," as used herein, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound provided herein will depend upon a number of factors including the isotopic purity of deuterated reagents used to make the compound and the efficiency of incorporation of deuterium in the various synthesis steps used to prepare the compound. However, as set forth above the relative amount of such isotopologues will be less than 49.9% of the compound.

A salt of a compound provided herein is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response, and are commensurate with a reasonable benefit/risk ratio. A "pharmaceutically acceptable salt" means any suitable salt that, upon administration to a recipient, is capable of providing, either directly or indirectly, a compound provided herein. A "pharmaceutically acceptable counterion" is an ionic portion of a salt that is not toxic when released from the salt upon administration to a recipient.
[1] Acids commonly employed to form pharmaceutically acceptable salts include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-l,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"Stereoisomer" refers to both enantiomers and diastereomers. "D" refers to deuterium. "Tert", " ^{t} ", and " t " each refer to tertiary. "US" refers to the United States of America. "RT" refers to room temperature. "h" refers to hours. "DMF" refers to dimethylformamide. "TsOH" refers to p-toluenesulfonic acid.

Throughout this specification, a variable may be referred to generally (e.g., "each R") or may be referred to specifically (e.g., R¹ or R²). Unless otherwise indicated, when a variable is referred to generally, it is meant to include all specific embodiments of that particular variable.

### Therapeutic Compositions and Methods

Described herein are combinations for use in the treatment of chronic or intractable pain in a subject in need thereof. The treatment comprises the administration of a deuterated dextromethorphan analog described herein and an antidepressant. In certain instances, the dextromethorphan analogs have enhanced metabolic profiles. The antidepressant is a selective serotonin reuptake inhibitor. The deuterated dextromethorphan analogs described herein and the antidepressant can be administered together in a single composition or administered in separate compositions.

The deuterated dextromethorphan analog is a compound of Formula **II:** 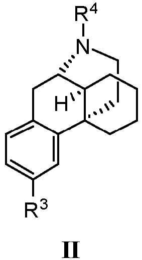 or a pharmaceutically acceptable salt thereof, wherein:
R³ is selected from -OCH₂D, -OCHD₂, and -OCD₃,; and
R⁴ is selected from -CH₃, -CH₂D, -CHD₂, and -CD₃;
provided that when R³ is -OCD₃, then R⁴ is not -CH₃.

In one embodiment, R⁴ is selected from -CH₂D, -CHD₂, and -CD₃. In a further embodiment, R³ is -OCD₃.

In one embodiment, R⁴ is -CH₃, -CHD₂ or -CD₃. In another embodiment, R⁴ is -CH₃. In another embodiment, R⁴ is -CD₃.

In yet another embodiment, the compound is selected from any one of the compounds set forth in Table 2.

**Table 2: Exemplary Compounds of Formula II**

| Compound No. | R³ | R⁴ |
|---|---|---|
| 112 | -OCD₃ | -CD₃ |
| 114 | -OCD₃ | -CHD₂ |

In another set of embodiments, any atom not designated as deuterium in any of the embodiments set forth above is present at its natural isotopic abundance.

In another set of embodiments, the compound of Formula **II** is purified, *e.g*., the compound of Formula **II** is present at a purity of at least 50.1% by weight (e.g., at least 52.5%, 55%, 60%, 65%, 70%, 75%, 80%, 85%, 90%, 95%, 97%, 98%, 98.5%, 99%, 99.5% or 99.9%) of the total amount of isotopologues of Formula **II** present, respectively. Thus, in some embodiments, a composition comprising a compound of Formula **II** can include a distribution of isotopologues of the compound, provided at least 50.1% of the isotopologues by weight are the recited compound.

In another set of embodiments, the compounds of Formula **II** are provided in isolated form, e.g., the compound is not in a cell or organism and the compound is separated from some or all of the components that typically accompany it in nature.

In some embodiments, any position in the compound of Formula **II** designated as having D has a minimum deuterium incorporation of at least 50.1% (e.g., at least 52.5%, at least 60%, at least 67.5%, at least 75%, at least 82.5%, at least 90%, at least 95%, at least 97%, at least 99%, or at least 99.5%) at the designated position(s) of the compound of Formula **II.** Thus, in some embodiments, a composition comprising a compound of Formula **II** can include a distribution of isotopologues of the compound, provided at least 50.1% of the isotopologues include a D at the designated position(s).

In some embodiments, a compound of Formula **II** is "substantially free of" other isotopologues of the compound, e.g., less than 49.9%, less than 25%, less than 10%, less than 5%, less than 2%, less than 1%, or less than 0.5% of other isotopologues are present.

The synthesis of compounds of Formula **II** can be readily achieved by synthetic chemists of ordinary skill by reference to the Exemplary Synthesis and Examples disclosed herein. Relevant procedures and intermediates are disclosed, for instance, in Schnider, O.; Grussner, A. Helv. Chim. Acta. 1951, 34, p 2211; Grussner, A. & Schnider, O.; GB 713146 (1954); Toyo Pharma K. K., Japan JP 60089474 A (1983); Newman, A. H. et al., J. Med. Chem. 1992, 35, p.4135. Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

The co-agent for inclusion in the combination for use in the methods is useful in the treatment of chronic or intractable pain, and is an antidepressant that is a selective serotonin reuptake inhibitor. In certain instances the co-agent is an inhibitor of a cytochrome p450 2D6 enzyme.

In certain instances the selective serotonin reuptake inhibitor can be citalopram, dapoxetine, escitalopram, fluvoxamine, norfluoxetine, fluoxetine, paroxetine, sertraline, or zimelidine, or pharmaceutically acceptable salts thereof.

### Exemplary Synthesis

The co-agents disclosed herein are commercially available or can be prepared using techniques known to those having ordinary skill in the art. Compounds of the disclosed dextromethorphan analog genuses can be prepared by a person skilled in the art using the appropriately deuterated reagents and/or intermediates according to the general procedures provided herein and described in the following publications and patents: (Schnider, O., Grussner, A., Helv. Chim. Acta. 1951, 34: 2211; Grussner, A., Schnider, O., GB 713146 (1954); Toyo Pharma K. K., Japan JP 60089474 A (1983); Newman, A. H. et al., J. Med. Chem. 1992, 35: 4135).

The following deuterated reagents and building blocks are commercially available: iodoethane-ds, ethyl-2,2,2-d₃ iodide, ethyl-1,1-d₂ iodide, isopropyl-d₇ iodide, isopropyl-d₇ bromide, isopropyl-1,1,1,3,3,3-d₆ iodide, and 1,1,1,3,3,3-d₆ bromide.

Compounds of Formula **II** may be prepared from one of the known intermediates **X, XI,** and **XII** shown below, and from related intermediates that may be readily obtained from known procedures.

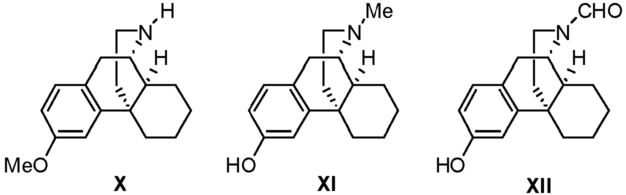

The scheme shown below shows a general route to the compounds of Formula **II.**

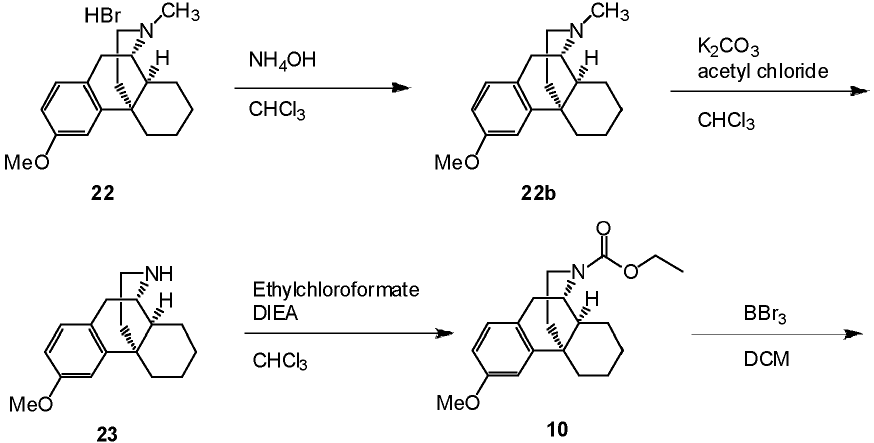

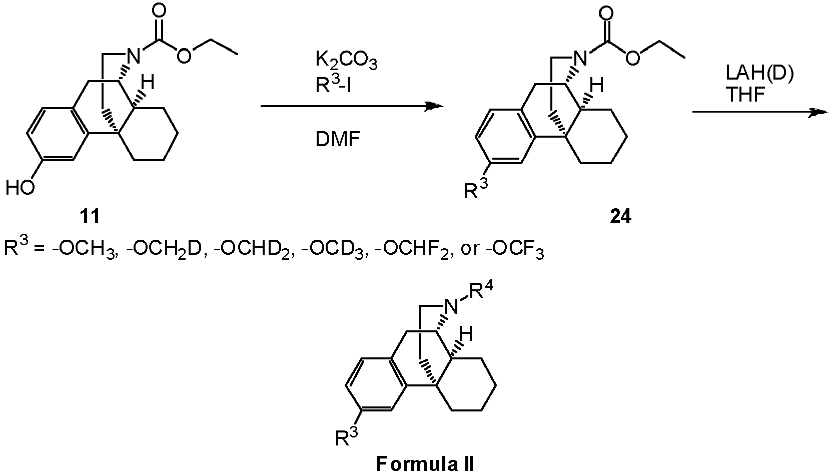

The scheme above shows a general route for preparing compounds of Formula **II.** The HBr salt, **22,** after treatment with NH₄OH produces free base **22b.** The free base **22b** is then *N*-demethylated via an acylative demethylation reaction followed by hydrolysis of the resulting acetamide to yield **23.** Acylation of the amine **23** using the ethylchloroformate provides the carbamate **10** which is then O-demethylated using BBr₃ to yield the alcohol **11.** Compound **11** is treated, in the presence of base, with an appropriately deuterated iodomethane to yield the ether **24,** which is reduced using either lithium aluminum deuteride (LAD) to yield compounds of Formula **II** wherein R⁴= -CD₃ or lithium aluminum hydride (LAH) to yield compounds of Formula **II** wherein R⁴= -CH₃. For those compounds of Formula **II** wherein R³ is -OCH₃, carbamate **10** is directly treated with LAD to produce a compound where R⁴ is -CD₃.

Various R³ groups (as defined in Formula **II**) may be introduced by O-alkylation of the appropriate phenol intermediate using an alkylating agent, such as an alkyl halide, according to methods generally known in the art. Various R⁴ groups (as defined in Formula **II**) may be introduced by *N*-alkylation using an R⁴-alkylating agent (for example, iodo-R⁴), or by reduction of the *N*-formyl group with a deuterated reagent, such as deuteroborane according to methods generally known in the art.

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹ or R²) or not. The suitability of a chemical group in a compound structure for use in the synthesis of another compound is within the knowledge of one of ordinary skill in the art.

Additional methods of synthesizing compounds of Formula **II** and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene TW et al., Protective Groups in Organic Synthesis, 3rd Ed., John Wiley and Sons (1999); Fieser L et al., Fieser and Fieser's Reagents for Organic Synthesis, John Wiley and Sons (1994); and Paquette L, ed., Encyclopedia of Reagents for Organic Synthesis, John Wiley and Sons (1995) and subsequent editions thereof.

### Compositions

Provided herein are pyrogen-free compositions comprising an effective amount of a compound of Formula **II** (e.g., including any of the formulae herein), or a pharmaceutically acceptable salt of said compound; a co-agent; and an acceptable carrier. In certain instances the composition is formulated for pharmaceutical use ("a pharmaceutical composition"), wherein the carrier is a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions provided herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds provided herein in pharmaceutical compositions may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound provided herein optionally formulated with a poloxamer, such as LUTROL^{™} and PLURONIC^{™} (BASF Corporation), or block copolymers of ethylene oxide and propylene oxide. See United States patent 7,014,866; and United States patent publications 20060094744 and 20060079502.

The pharmaceutical compositions provided herein include those suitable for oral, rectal, nasal, topical (including buccal and sublingual), vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington: The Science and Practice of Pharmacy, Lippincott Williams & Wilkins, Baltimore, MD (20th ed. 2000).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In certain embodiments, the compounds are administered orally. The compositions provided herein suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,₃-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions provided herein may be administered in the form of suppositories for rectal administration. These compositions can be prepared by mixing a compound provided herein with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions provided herein can be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g.: Rabinowitz JD and Zaffaroni AC, US Patent 6,803,031, assigned to Alexza Molecular Delivery Corporation.

Topical administration of the pharmaceutical compositions provided herein are useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds provided herein include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and water. The pharmaceutical compositions provided herein may also be topically applied to the lower intestinal tract by rectal suppository formulation or in a suitable enema formulation.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access.

Thus, according to yet another embodiment, the compounds provided herein may be incorporated into compositions for coating an implantable medical device, such as prostheses, artificial valves, vascular grafts, stents, or catheters. Suitable coatings and the general preparation of coated implantable devices are known in the art and are exemplified in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, provided is a method of coating an implantable medical device comprising the step of contacting said device with the coating composition described above. It will be obvious to those skilled in the art that the coating of the device will occur prior to implantation into a mammal.

According to another embodiment, provided is a method of impregnating an implantable drug release device comprising the step of contacting said drug release device with a compound or composition provided herein. Implantable drug release devices include, but are not limited to, biodegradable polymer capsules or bullets, non-degradable, diffusible polymer capsules and biodegradable polymer wafers.

According to another embodiment, provided is an implantable medical device coated with a compound or a composition comprising a compound provided herein, such that said compound is therapeutically active.

According to another embodiment, provided is an implantable drug release device impregnated with or containing a compound or a composition comprising a compound provided herein, such that said compound is released from said device and is therapeutically active.

Where an organ or tissue is accessible because of removal from the subject, such organ or tissue may be bathed in a medium containing a composition provided herein, a composition provided herein may be painted onto the organ, or a composition provided herein may be applied in any other convenient way.

In another embodiment, provided are separate dosage forms of a compound of Formula **II;** and one or more of any of the above-described co-agents, wherein the compound of Formula **II;** and the co-agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions provided herein, the compound of Formula **II** is present in an effective amount. As used herein, the term "effective amount" refers to an amount which, when administered in a proper dosing regimen, is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy.

The interrelationship of dosages for animals and humans (based on milligrams per meter squared of body surface) is described in Freireich et al., (1966) Cancer Chemother. Rep 50: 219. Body surface area may be approximately determined from height and weight of the subject. See, e.g., Scientific Tables, Geigy Pharmaceuticals, Ardsley, N.Y., 1970, 537.

In one embodiment, an effective amount of a compound of Formula **II** can range from 0.4 mg to 400 mg, from 4.0 mg to 350 mg, from 10 mg to 90 mg, or from 30 mg to 45 mg, inclusive, which can be given once, twice, or up to three times daily depending on various factors recognized by those skilled in the art.

Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician. For example, guidance for selecting an effective dose can be determined by reference to the prescribing information for dextromethorphan.

An effective amount of the co-agent can be between about 0.01% to about 100% of the dosage normally utilized in a monotherapy regime using only that agent. The normal monotherapeutic dosages of these co-agents are well known in the art. *See,* e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000).

### Methods of Treatment

Provided herein is a method of treating chronic or intractable pain. Chronic or intractable pain includes pain related to stroke, trauma, cancer, cancer treatment, fibromyalgia, and pain due to neuropathies such as herpes zoster infection (*i.e*., postherpetic neuralgia), and diabetes (diabetic neuropathy). Neuropathic pain also includes phantom limb pain, trigeminal neuralgia, and sciatica. The method comprises the step of administering to a subject a therapeutically effective amount of a co-agent and a deuterated dextromethorphan analog. The co-agent is an antidepressant that is a selective serotonin reuptake inhibitor; or pharmaceutically acceptable salts thereof. The deuterated dextromethorphan analog is a compound of Formula **II:** 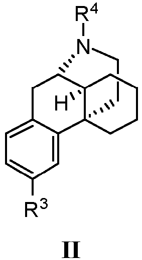 or a pharmaceutically acceptable salt thereof, wherein:
R³ is selected from -OCH₂D, -OCHD₂, and -OCD₃; and
R⁴ is selected from -CH₃, -CH₂D, -CHD₂, and -CD₃;
provided that when R³ is -OCD₃, then R⁴ is not -CH₃.

In certain embodiments R⁴ is -CH₃, -CHD₂, or -CD₃. In certain embodiments R³ is -OCD₃.

In some instances, the co-agent described above is capable of inhibiting the action of a cytochrome p450 2D6 enzyme.

The co-agent is a selective serotonin reuptake inhibitor. The selective serotonin reuptake inhibitor can be fluoxetine, norfluoxetine, citalopram, dapoxetine, escitalopram, fluvoxamine, paroxetine, or sertraline, or pharmaceutically acceptable salts thereof. In certain instances the co-agent is citalopram, norfluoxetine, dapoxetine, escitalopram, fluvoxamine, paroxetine, or sertraline, or pharmaceutically acceptable salts thereof. In another embodiment the co-agent is paroxetine, or a pharmaceutically acceptable salt thereof.

Also provided is use in a method of treating chronic or intractable pain in a subject in need thereof, the method comprising the step of administering to the subject a therapeutically effective amount of a co-agent selected from the grouping consisting of citalopram, fluvoxamine, norfluoxetine, fluoxetine, paroxetine, sertraline, or pharmaceutically acceptable salts thereof, and a therapeutically effective amount of a compound selected from the group consisting of: 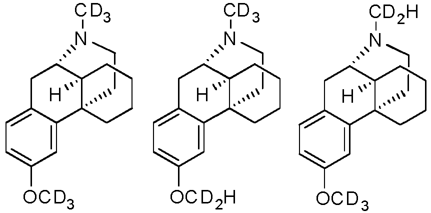 or pharmaceutically acceptable salts thereof.

Another embodiment relates to the aforementioned use in a method where in the co-agent can be citalopram, fluvoxamine, paroxetine, sertraline, or pharmaceutically acceptable salts thereof. The co-agent can also be paroxetine, or a pharmaceutically acceptable salt thereof.

Another embodiment relates to any of the aforementioned use in methods for treating chronic or intractable pain, where the chronic or intractable pain is a neuropathic pain.

Another embodiment relates to any of the aforementioned use in methods for treating chronic or intractable pain, where the chronic or intractable pain is diabetic neuropathic pain.

The co-agent may be administered together with a compound of Formula **II** as part of a single dosage form or as separate, multiple dosage forms. Alternatively, the co-agent may be administered prior to, consecutively with, or following the administration of a compound of Formula **II.** In such combination therapy treatment, both the compound of Formula **II;** and the co-agent are administered by conventional methods.

Effective amounts of the co-agent are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the co- agent's optimal effective-amount range.

In certain embodiments, the effective amount of the co-agent is less than its effective amount would be where the compound of Formula **II** is not administered. In this way, undesired side effects associated with high doses of either agent may be minimized. Other potential advantages (including without limitation improved dosing regimens and/or reduced drug cost) will be apparent to those of skill in the art.

In one embodiment, the therapeutically effective amount of the compound of Formula II is lower than the therapeutically effective amount of dextromethorphan that is sufficient to achieve the same therapeutic effect as the compound for Formula II.

In one embodiment, a therapeutically effective amount of the compound of Formula II is administered with a co-agent wherein the amount of the co-agent is less than its amount would be where the co-agent is administered in the absence of the compound of Formula II and with an amount of dextromethorphan equal to the therapeutically effective amount of the compound of Formula II. In this way, undesired side effects associated with high doses of the co-agent may be minimized. In an example of this embodiment, the co-agent is quinidine or a pharmaceutically acceptable salt thereof, such as quinidine sulfate.

In one aspect of this embodiment, an amount of the compound of Formula **II** or a pharmaceutically acceptable salt thereof, such as the hydrobromide salt, wherein the amount is between about 10 mg and about 45 mg, such as between about 7.5 mg and about 30 mg, is administered with (a) an amount of quinidine sulfate between about 1 mg and about 25 mg, such as between about 2.5 mg and about 15 mg, or (b) an amount of quinidine or of a pharmaceutically acceptable salt thereof other than quinidine sulfate that is equimolar with an amount of quinidine sulfate between about 1 mg and about 25 mg, such as between about 2.5 mg and about 15 mg. As an example, the compound of Formula II or a pharmaceutically acceptable salt thereof, such as the hydrobromide salt, and the quinidine sulfate, quinidine, or pharmaceutically acceptable salt thereof other than quinidine sulfate may be administered in the therapeutically effective amounts above to a subject to treat diabetic neuropathy or neuropathic pain such as diabetic neuropathic pain.

In another aspect of this embodiment, an amount of the compound of Formula Formula II or a pharmaceutically acceptable salt thereof, such as the hydrobromide salt, wherein the amount is between about 10 mg and about 45 mg, such as between about 7.5 mg and about 30 mg, is administered with an amount of (a) an amount of quinidine sulfate between about 1 mg and about 25 mg, such as between about 1 mg and about 7.5 mg, or (b) an amount of quinidine or of a pharmaceutically acceptable salt thereof other than quinidine sulfate that is equimolar with an amount of quinidine sulfate between about 1 mg and about 25 mg, such as between about 1 mg and about 7.5 mg.

Certain embodiments relate to any of the aforementioned methods, where an effective amount of a compound of Formula **II** can range from about 0.4 mg to about 400 mg, from about 4.0 mg to about 350 mg, from about 10 mg to about 250 mg, from about 10 mg to about 150 mg, from about 10 mg to about 90 mg, from about 1 mg to about 60 mg, from about 10 mg to about 40 mg, from about 20 mg to about 30 mg, or from about 30 mg to about 45 mg. The dose be given once, twice, or up to three times daily depending on various factors recogonized by those skilled in the art.

Certain embodiments relate to any of the aforementioned methods, where the co-agent is paroxetine. In certain instances, an effective amount of paroxetine, when dosed with a compound of Formula **II,** can range from about 1 mg to about 40 mg, from about 1 mg to about 30 mg, from about 5 mg to about 40 mg, from about 5 mg to about 25 mg, from about 10 mg to about 40 mg, from about 10 mg to about 20 mg, from about 15 mg to about 40 mg, from about 20 mg to about 40 mg, from about 20 mg to about 35 mg, or from about 25 mg to about 35 mg.

In yet another aspect, provided is the use of a compound of Formula **II** together with one or more of the above-described co-agents in the manufacture of a medicament, either as a single composition or as separate dosage forms, for treatment or prevention in a subject of a disease, disorder or symptom set forth above.

### Kits

Also provided are kits for use to treat chronic or intractable pain. These kits comprise (a) a pharmaceutical composition comprising a compound of Formula **II** and a co-agent, as described above, or pharmaceutically acceptable salts thereof, wherein said pharmaceutical composition is in a container; and (b) instructions describing a method of using the pharmaceutical composition to treat chronic or intractable pain.

In certain embodiments, the kits comprise (a) a first pharmaceutical composition comprising a compound of Formula **II** or pharmaceutically acceptable salts thereof; (b) a second pharmaceutical composition comprising a co-agent as described above or a pharmaceutically acceptable salt thereof, wherein the first pharmaceutical composition and the second pharmaceutical composition are contained in separate containers; and (c) instructions describing a method of using the first pharmaceutical composition and the second pharmaceutical composition to treat chronic or intractable pain.

The container(s) may be any vessel or other sealed or sealable apparatus that can hold said pharmaceutical composition(s). Examples include bottles, ampules, divided or multi-chambered holders bottles, wherein each division or chamber comprises a single dose of said composition, a divided foil packet wherein each division comprises a single dose of said composition, or a dispenser that dispenses single doses of said composition. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box. In on embodiment, the container is a blister pack.

The kits may also comprise a device to administer or to measure out a unit dose of the pharmaceutical composition. Such device may include an inhaler if said composition is an inhalable composition; a syringe and needle if said composition is an injectable composition; a syringe, spoon, pump, or a vessel with or without volume markings if said composition is an oral liquid composition; or any other measuring or delivery device appropriate to the dosage formulation of the composition present in the kit.

### Examples

**Example 1 (reference).** Synthesis of (+)-3-(Ethoxy-d₅)-17-(methyl-d₃)-(9α,13α,14α)-morphinan hydrochloride **(100).**

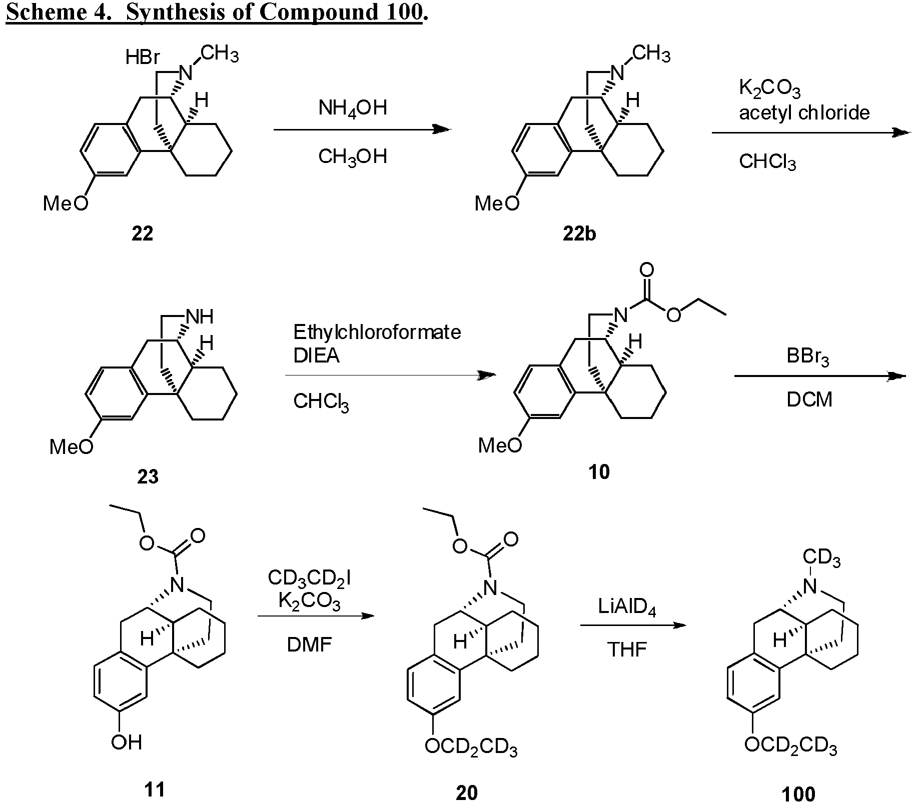

**Synthesis of (+)-3-methoxy-17-methyl-(9α,13α,14α)-morphinan (free base,** 22b). To a reaction vessel was added (+)-3-methoxy-17-methyl-(9α,13α,14α)-morphinan, HBr salt (22; 3.00g, 8.5 mmol), NH₃ in CH₃OH (2.0 M, 8.5 mL, 17.0 mmol), and a stir bar. The reaction mixture was stirred at RT for 1 h. The resulting material was concentrated on a rotary evaporator, then diluted with CHCl₃ (50 mL) and H₂O (50 mL). The layers were separated and the water layer was extracted with CHCl₃ (50 mL). The combined organic layers were dried over magnesium sulfate, filtered and concentrated on a rotary evaporator to yield 2.88 g of **22b** as a fluffy white solid.

**¹H-NMR** (300 MHz, CDCl₃): δ 1.12 (ddd, *J₁*=24.7, *J₂*=12.6, *J₃*=3.8, 1H), 1.23-1.43 (m, 5H), 1.49-1.52 (m, 1H), 1.62-1.65 (m, 1H), 1.72 (td, *J₁*=12.6, *J₂*=4.9, 1H), 1.81 (dt, *J₁*=12.6, *J₂*=3.3, 1H), 2.07 (td, *J₁=*12.6, *J₂*=3.3, 1H), 2.33-2.47 (m, 5H), 2.57 (dd, *J₁*=18.1, *J₂=*5.5, 1H), 2.79 (dd, *J₁*=5.5, *J₂*=3.3, 1H), 2.98 (d, *J*=18.1, 1H), 6.68 (dd, *J₁*=8.2, *J₂*=2.7, 1H), 6.80 (d, *J*=2.7, 1H), 7.02 (d, J=8.8, 1H).

**Synthesis of (+)-3-methoxy-(9α,13α,14α)-morphinan (23).** The solid (+)-3-methoxy-17-methyl-(9α,13α,14α)-morphinan (**22b**; 6.79 g, 25.1 mmol) was placed in a reaction vessel with CHCl₃ and a stir bar. K₂CO₃ (13.85 g, 100.2 mmol) was added and the mixture was stirred at RT under an atmosphere of N₂ for 10 min before the addition of acetyl chloride (7.866 g, 100.2 mmol). The resulting reaction mixture, still under an atmosphere of N₂, was stirred under reflux conditions for 7 h, then filtered through a pad of celite. The organic filtrate was concentrated on a rotary evaporator and the resulting crude material was dissolved in CH₃OH then stirred under reflux conditions for 1 h. The solution was concentrated on a rotary evaporator then dried under vacuum to yield 6.78 g of **23** as an off-white solid.

**¹H-NMR** (300 MHz, CDCl₃): δ 1.04-1.13 (m, 1H), 1.19-1.29 (m, 1H), 1.37-1.66 (m, 6H), 2.37 (d, *J*=13.5, 2H), 2.54 (bs, 1H), 2.80 (s, 2H), 2.95-2.99 (m, 1H), 3.12-3.18 (m, 2H), 3.48 (s, 1H), 3.71 (s, 3H), 6.76 (dd, *J₁*=8.3, *J₂*=2.6, 1H), 6.80 (d, *J*=2.3, 1H), 7.07 (d, *J*=8.3, 1H).

**Synthesis of (+)-17-ethylcarbamate-3-methoxy-(9α,13α,14α)-morphinan (10).** To a reaction vessel fit with a stirbar was added 23 (6.025g, 2.48 mmol) dissolved in CHCl₃ (100 mL). Diisopropylethylamine (DIEA; 16.32 g, 126.3 mmol) was added and the mixture was stirred for 10 min at room temperature under nitrogen before the addition of ethylchloroformate (13.094 g, 76.8 mmol). The reaction mixture was stirred under reflux conditions under nitrogen for 3 h, at which point TLC (20% ethylacetate/hexane) showed complete consumption of the starting material. The organic layer was removed and washed first with 1M HCl, and then with saturated NaHCO₃. The aqueous layers from each wash were combined and back extracted with 50 ml of CHCl₃. The organic layer from the back extraction was combined with the organic layer from the washes and the combined organic layers were dried over Na₂SO₄. The organic solution was then filtered, concentrated on a rotary evaporator then was purified via automated flash column chromatography (0-30% ethylacetate/hexane) to yield 5.37 g of **10** as a clear light yellow oil.

**¹H-NMR** (300 MHz, CDCl₃): δ 1.06 (ddd, *J₁*=25.3, *J₂*=12.6, *J₃*=3.8, 1H), 1.21-1.39 (m, 7H), 1.45-1.60 (m, 3H), 1.65-1.70 (m, 2H), 2.34-2.37 (m, 1H), 2.54-2.69 (m, 2H), 3.04-3.12 (m, 1H), 3.78 (s, 3H), 3.86 (ddd, *J₁*=42.3*, J₂*=13.7, *J₃*=3.8, 1H), 4.12 (q, *J*=7.14, 2H), 4.31 (dt, *J₁*=56.6, *J₂*=4.3, 1H), 6.71 (dd, *J₁*=8.8, *J₂=2.2,* 1H), 6.82 (d, *J*=2.7, 1H), 7.00 (apparent t, *J*=8.2, 1H).

**(+)-17-ethylcarbamate-3-hydroxy-(9α,13α,14α)-morphinan (11).** In a reaction vessel fit with a stirbar the carbamate **10** (2.43 g, 7.4 mmol) was dissolved in DCM (20 mL) and the resulting solution was cooled to 0 °C. BBr₃ (9.24 g, 36.9 mmol) was added and the reaction mixture was stirred under an atmosphere of N₂ at 0 °C for 20 min (at which time tlc in 20% ethylacetate/hexane showed the reaction to be complete). A solution of 27% NH₄OH in ice was placed in a beaker with a stir bar and the reaction mixture was slowly added with stirring. The resulting mixture was stirred for 20 min then was extracted with 4:1 CHCl₃/CH₃OH (200 mL). The organic layer was dried over Na₂SO₄, filtered, then concentrated on a rotary evaporator. The crude material was purified via automated flash column chromatography (CH₃OH with 1% NH₄OH / CHCl₃, 0-10%). The pure fractions were concentrated on a rotary evaporator to yield 1.48 g of **11** as a white solid.

**¹H-NMR** (300 MHz, CDCl₃): δ 1.04-1.12 (m, 1H), 1.22-1.36 (m, 7H), 1.45-1.59 (m, 3H), 1.63-1.67 (m, 2H), 2.30-2.33 (m, 1H), 2.52-2.66 (m, 2H), 3.06 (dt, *J₁*=18.4, *J₂*=5.9, 1H), 3.84 (ddd, *J₁*=35.8, *J₂*=13.8, *J₃*=6.1, 1H), 4.10-4.18 (m, 2H), 4.31 (dt, *J₁*=53.9, *J₂*=3.1, 1H), 6.64 (m, 1H), 6.78 (s, 1H), 6.93 (apparent t, *J*=7.8, 1H).

**Synthesis of (+)-3-(ethoxy-d₅)-17-ethoxycarbonyl-(9α,13α,14α)-morphinan (20).** To a solution of alcohol **11** (1.50 g, 4.8 mmol) in DMF (25 mL), was added K₂CO₃ (2.00 g, 14.5 mmol, 3.05 eq) and iodoethane-ds (1.15 g, 7.1 mmol, 1.50 eq) with stirring. The reaction mixture was stirred overnight at room temperature under an atmosphere of N₂, was quenched by the addition of H₂O, and extracted with Et₂O (3 x 30 mL). The combined organics were dried over Na₂SO₄, filtered and concentrated *in vacuo* to a yellow oil. Purification via automated flash column chromatography (0-40% EtOAc/hexanes) afforded intermediate **20** (1.53 g, 91% yield).

**Synthesis of (+)-3-(ethoxy-d₅)-17-(methyl-d₃)-(9α,13α,14α)-morphinan hydrochloride (100).** To a slurry of LiAlD₄ (0.184 g, 4.4 mmol, 2.0 eq) in THF (10 mL) stirring at -78 °C was added a solution of the carbamate **20** (0.763 g, 2.2 mmol) in THF (5 mL). After 1 h of stirring at rt, no reaction was detected by tlc and an additional 2.0 eq of LiAlD₄ (0.184 g, 4.4 mmol, 2.0 eq) was added. The reaction mixture was stirred overnight at rt, then was quenched by the addition of magnesium sulfate heptahydrate until cessation of gas evolution. The mixture was filtered, concentrated *in vacuo* and the resultant crude material was purified via automated flash column chromatography (CHCl₃/CH₃OH/NH₃OH - 90/10/1) to yield the free amine **100.** This material was dissolved in 1.25 M HCl in CH₃OH then was concentrated under reduced pressure and dried under high vacuum to yield 14.3 mg of product 100 as the HCl salt. **¹H-NMR** (300 MHz, DMSO-d₆): δ 0.94-1.63 (m, 8H), 1.72-1.80 (m, 1H), 1.94 (d, *J*=11.9, 1H), 2.43-2.47 (m, 1H), 2.96 (dd, *J₁*=19.2, *J₂*=6.1, 2H), 3.09-3.17 (m, 2H), 3.57-3.61 (m, 1H), 6.79-6.82 (m, 2H), 7.11 (d, *J*=8.8, 1H), 9.58 (br s, 1H). **HPLC** (method: 150 mm C18-RP column - gradient method 5-95% ACN; Wavelength: 280 nm): retention time: 3.08 min, purity: 95%. **MS** (M+H): 294.2.

**Example 2 (reference).** Synthesis of (+)-3-(Ethoxy-d₅)-17-methyl-(9α,13α,14α)-morphinan hydrochloride **(104).**₄ was used in place of LiAlD₄ for the reduction of the carbamate **20** to **104.**

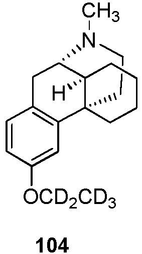

**Synthesis of (+)-3-(ethoxy-d₅)-17-methyl-(9α,13α,14α)-morphinan hydrochloride (104).** To a slurry of LiAlH₄ (0.166 g, 4.4 mmol, 2.0 eq) in THF (10 mL) stirring at -78 °C was added a solution of the carbamate **20** (0.763 g, 2.2 mmol) in THF (5 mL). After 1 h an additional 2.0 eq of LiAlH₄ (0.184 g, 4.4 mmol, 2.0 eq) was added. The reaction mixture was stirred overnight at rt, then was quenched by the addition of magnesium sulfate heptahydrate until cessation of gas evolution. The mixture was filtered, concentrated *in vacuo* and the resultant crude material was purified via automated flash column chromatography (CHCl₃/CH₃OH/NH₃OH - 90/10/1) to yield the free-amine **104.** This material was dissolved in 1.25 M HCl in CH₃OH then was concentrated under reduced pressure and dried under high vacuum to yield 31 mg of product **104** as the HCl salt. **¹H-NMR** (300 MHz, DMSO-d₆): δ 0.94-1.64 (m, 8H), 1.74-1.82 (m, 1H), 1.97 (d, *J*=12.4, 1H), 2.44-2.47 (m, 1H), 2.81 (s, 3H), 2.96 (dd, *J₁*=20.0, *J₂*=5.8, 2H), 3.09-3.18 (m, 2H), 3.55-3.62 (m, 1H), 6.79-6.82 (m, 2H), 7.12 (d, *J*=9.1, 1H), 9.68 (br s, 1H). **HPLC** (method: 150 mm C18-RP column - gradient method 5-95% ACN; Wavelength: 280 nm): retention time: 3.00 min, purity: 95%. **MS** (M+H): 291.2.

**Example 3 (reference).** Synthesis of (+)-3-(Isopropoxy-d₇)-17-(methyl-d₃)-(9α,13α,14α)-morphinan **(102).**

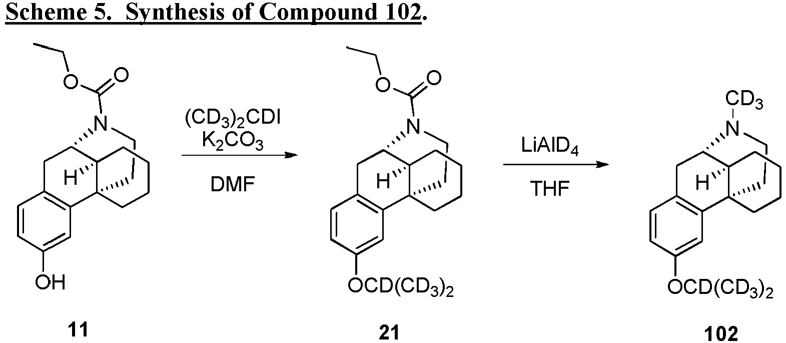

**Synthesis of (+)-3-(isopropoxy-d₇)-17-ethoxycarbonyl-(9α,13α,14α)-morphinan (21).** To a solution of alcohol **11** (1.50 g, 4.8 mmol; produced according to Example 1) in DMF (25 mL), was added K₂CO₃ (2.00 g, 14.5 mmol, 3.05 eq) and 2-iodopropane-d₇ (0.71 mL, 7.1 mmol, 1.50 eq) with stirring. The reaction mixture was stirred overnight at room temperature under an atomosphere of N₂, was quenched by the addition of H₂O, and extracted with Et₂O (3 x 30 mL). The combined organics were dried over Na₂SO₄, filtered and concentrated *in vacuo* to a colorless oil. Purification via automated flash column chromatography (0-40% EtOAc/hexanes) afforded intermediate **21** (1.48 g, 85% yield).

**Synthesis of (+)-3-(isopropoxy-d₇)-17-(methyl-d₃)-(9α,13α,14α)-morphinan (102).** To a slurry of LiAlD₄ (0.340 g, 8.1 mmol, 4.0 eq) in THF (10 mL) stirring at -78 °C was added a solution of the carbamate **21** (0.739 g, 2.0 mmol) in THF (5 mL). The reaction mixture was stirred overnight at rt, then was quenched by the addition of magnesium sulfate heptahydrate until cessation of gas evolution. The mixture was filtered, the filtrate concentrated *in vacuo* and the resultant material was dissolved in CH₃OH. The resulting solution was acidified to pH 4 with fumaric acid resulting in salt precipitation. The mixture was stirred for 5 min, and Et₂O was added to bring remaining salt out of solution. The salt was isolated by filtration and dried to yield 660 mg of final product **102** as the fumaric acid salt. **¹H-NMR** (300 MHz, CDCl₃): δ 1.10 (qd, *J₁*=12.6, *J₂*=3.8, 1H), 1.21-1.68 (m, 7H), 2.01 (td, *J₁*=13.6, *J₂*=4.5, 1H), 2.16-2.21 (m, 1H), 2.32-2.47 (m, 2H), 2.99-3.01 (m, 2H), 3.10-3.13 (m, 1H), 3.44-3.46 (m, 1H), 6.72 (dd, *J₁*=8.4, *J₂*=2.4, 1H), 6.79 (d, *J*=2.5, 1H), 6.82 (s, 1H), 7.03 (d, *J*=8.3, 1H). **HPLC** (method: 150 mm C18-RP column - gradient method 5-95% ACN; Wavelength: 280 nm): retention time: 3.11 min, purity: 95%. **MS** (M+H): 310.3.

**Example 4 (reference).** Synthesis of (+)-3-(Isopropoxy-d₇)-17-methyl-(9α,13α,14α)-morphinan **(106).**₄ was used in place of LiAlD₄ for the reduction of the carbamate 21 to **106.**

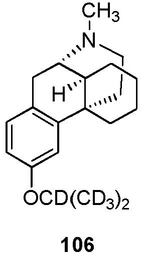

**Synthesis of (+)-3-(isopropoxy-d₇)-17-methyl-(9α,13α,14α)-morphinan (106).** To a slurry of LiAlH₄ (0.308 g, 8.1 mmol, 4.0 eq) in THF (10 mL) stirring at -78 °C was added a solution of the carbamate **21** (0.739 g, 2.0 mmol) in THF (5 mL). The reaction mixture was stirred overnight at rt, then was quenched by the addition of magnesium sulfate heptahydrate until cessation of gas evolution. The mixture was filtered, the filtrate concentrated *in vacuo* and the resultant material was dissolved in CH₃OH. The resulting solution was acidified to pH 4 with fumaric acid resulting in salt precipitation. The mixture was stirred for 5 min, and Et₂O was added to bring remaining salt out of solution. The salt was isolated by filtration and dried to yield 330 mg of final product **106** as the fumaric acid salt. **¹H-NMR** (300 MHz, CDCl₃): δ 1.09 (qd, *J₁*=12.6*, J₂*=3.8*,* 1H), 1.22-1.58 (m, 6H), 1.65 (d, *J*=12.6, 1H), 2.06 (td, *J₁*=13.5, *J₂*=4.3, 1H), 2.20 (d, *J*=12.4, 1H), 2.35 (d, *J*=13.3, 1H), 2.46-2.53 (m, 1H), 2.78 (s, 3H), 2.96-3.12 (m, 2H), 3.25-3.30 (m, 1H), 3.62-3.64 (m, 1H), 6.73 (dd, *J₁*=8.3, *J₂*=2.5, 1H), 6.80 (d, *J*=2.5, 1H), 6.86 (s, 2H), 7.05 (d, *J*=8.3, 1H). **HPLC** (method: 150 mm C18-RP column - gradient method 5-95% ACN; Wavelength: 280 nm): retention time: 3.18 min, purity: 95%. **MS** (M+H): 307.4.

**Example 5.** Synthesis of (+)-3-(Methoxy-d₃)-17-(methyl-d₃)-(9α,13α,14α)-morphinan **(112).** 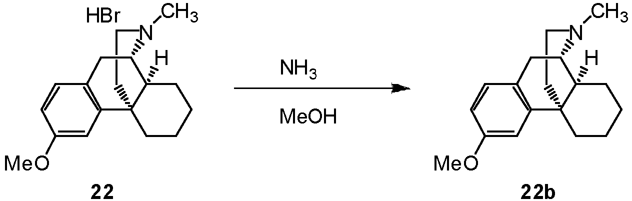 *(+)-3-methoxy-17-methyl-(9α, 13α, 14*α*)-morphinan (**22b**).* To a reaction vessel was added (+)-3-methoxy-17-methyl-(9α,13α,14α)-morphinan, HBr salt **22** (3.00g, 8.5 mmol), NH₃ in CH₃OH (2.0 M, 8.5 mL, 17.0 mmol), and a stir bar. The reaction mixture was stirred at RT for 1 h. The resulting material was concentrated on a rotary evaporator, then diluted with CHCl₃ (50 mL) and H₂O (50 mL). The layers were separated and the water layer was extracted with CHCl₃ (50 mL). The combined organic layers were dried over magnesium sulfate, filtered and concentrated on a rotary evaporator to yield 2.88 g of **22b** as a fluffy white solid.

**¹H-NMR** (300 MHz, CDCl₃): δ 1.12 (ddd, *J₁*=24.7, *J₂*=12.6, *J₃*=3.8, 1H), 1.23-1.43 (m, 5H), 1.49-1.52 (m, 1H), 1.62-1.65 (m, 1H), 1.72 (td, *J₁*=12.6, *J₂*=4.9, 1H), 1.81 (dt, *J₁*=12.6, *J₂*=3.3, 1H), 2.07 (td, *J₁*=12.6, *J₂*=3.3, 1H), 2.33-2.47 (m, 5H), 2.57 (dd, *J₁*=18.1, *J₂*=5.5, 1H), 2.79 (dd, *J₁*=5.5, *J₂*=3.3, 1H), 2.98 (d, *J*=18.1, 1H), 6.68 (dd, *J₁*=8.2, *J₂*=2.7, 1H), 6.80 (d, *J*=2.7, 1H), 7.02 (d, J=8.8, 1H).

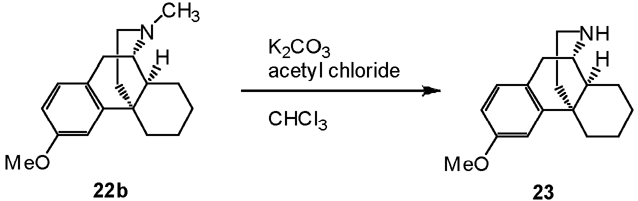

*(+)-3-methoxy-(9α,13α,14α)-morphinan **(23).*** The solid **22b** (6.79 g, 25.1 mmol) was placed in a reaction vessel with CHCl₃ and a stir bar. K₂CO₃ (13.85 g, 100.2 mmol) was added and the mixture was stirred at RT under an atmosphere of N₂ for 10 min before the addition of acetyl chloride (7.866 g, 100.2 mmol). The resulting reaction mixture, still under an atmosphere of N₂, was stirred under reflux conditions for 7 h, then filtered through a pad of celite. The organic filtrate was concentrated on a rotary evaporator and the resulting crude material was dissolved in CH₃OH then stirred under reflux conditions for 1 h. The solution was concentrated on a rotary evaporator then dried under vacuum to yield 6.78 g of **23** as an off-white solid.

**¹H-NMR** (300 MHz, CDCl₃): δ 1.04-1.13 (m, 1H), 1.19-1.29 (m, 1H), 1.37-1.66 (m, 6H), 2.37 (d, J=13.5, 2H), 2.54 (bs, 1H), 2.80 (s, 2H), 2.95-2.99 (m, 1H), 3.12-3.18 (m, 2H), 3.48 (s, 1H), 3.71 (s, 3H), 6.76 (dd, *J₁*=8.3, *J₂*=2.6, 1H), 6.80 (d, *J* =2.3, 1H), 7.07 (d, *J* =8.3, 1H).

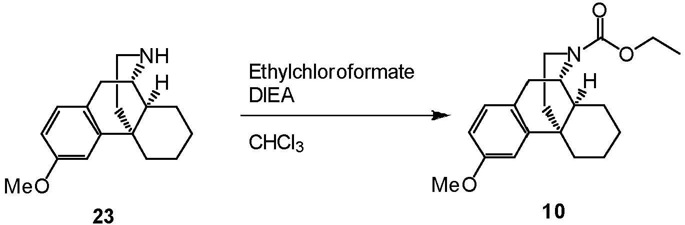

*(+)-17-ethylcarbamate-3-methoxy-(9α,13α,14α)-morphinan **(10).*** To a reaction vessel fit with a stirbar was added **23** (6.025g, 2.48 mmol) dissolved in CHCl₃ (100 mL). Diisopropylethylamine (DIEA; 16.32 g, 126.3 mmol) was added and the mixture was stirred for 10 min at room temperature under nitrogen before the addition of ethylchloroformate (13.094 g, 76.8 mmol). The reaction mixture was stirred under reflux conditions under nitrogen for 3 h, at which point tlc (20% ethylacetate/hexane) showed complete consumption of starting material, **23.** The organic layer was removed and washed first with 1M HCl, and then with saturated NaHCO₃. The aqueous layers from each wash were combined and back extracted with 50 mL of CHCl₃. The organic layer from the back extraction was combined with the organic layer from the washes and the combined organic layers were dried over NaSO₄. The organic solution was then filtered, concentrated on a rotary evaporator then was purified via automated flash column chromatography (0-30% ethylacetate/hexane) to yield 5.37 g of 10 as a clear light yellow oil.

**¹H-NMR** (300 MHz, CDCl₃): δ 1.06 (ddd, *J₁*=25.3, *J₂*=12.6, *J₃*=3.8, 1H), 1.21-1.39 (m, 7H), 1.45-1.60 (m, 3H), 1.65-1.70 (m, 2H), 2.34-2.37 (m, 1H), 2.54-2.69 (m, 2H), 3.04-3.12 (m, 1H), 3.78 (s, 3H), 3.86 (ddd, *J₁*=42.3, *J₂*=13.7, *J₃*=3.8, 1H), 4.12 (q, *J*=7.14, 2H), 4.31 (dt, *J₁*=56.6, *J₂*=4.3, 1H), 6.71 (dd, *J₁*=8.8, *J₂*=2.2, 1H), 6.82 (d, *J* =2.7, 1H), 7.00 (apparent t, *J* =8.2, 1H).

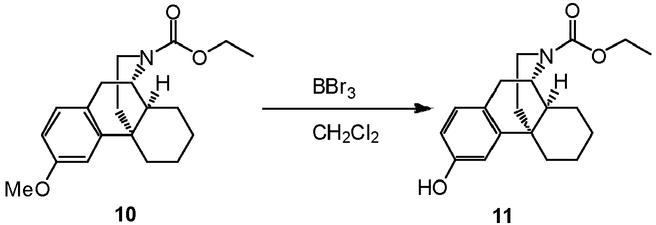

*(+)-17-ethylcarbamate-3-hydroxy-(9α,13α,14α)-morphinan **(11).*** In a reaction vessel fit with a stirbar the carbamate **10** (2.43 g, 7.4 mmol) was dissolved in DCM (20 mL) and the resulting solution was cooled to 0 °C. BBr₃ (9.24 g, 36.9 mmol) was added and the reaction mixture was stirred under an atmosphere of N₂ at 0 °C for 20 min (at which time tlc in 20% ethylacetate/hexane showed the reaction to be complete). A solution of 27% NH₄OH in ice was placed in a beaker with a stir bar and the reaction mixture was slowly added with stirring. The resulting mixture was stirred for 20 min then was extracted with 4:1 CHCl₃/CH₃OH (200 mL). The organic layer was dried over Na₂SO₄, filtered, then concentrated on a rotary evaporator. The crude material was purified via automated flash column chromatography (CH₃OH with 1% NH₄OH / CHCl₃, 0-10%). The pure fractions were concentrated on a rotary evaporator to yield 1.48 g of **11** as a white solid.

**¹H-NMR** (300 MHz, CDCl₃): δ 1.04-1.12 (m, 1H), 1.22-1.36 (m, 7H), 1.45-1.59 (m, 3H), 1.63-1.67 (m, 2H), 2.30-2.33 (m, 1H), 2.52-2.66 (m, 2H), 3.06 (dt, *J₁*=18.4, *J₂*=5.9, 1H), 3.84 (ddd, *J₁*=35.8, *J₂*=13.8, *J₃*=6.1*,* 1H), 4.10-4.18 (m, 2H), 4.31 (dt, *J₁*=53.9, *J₂*=3.1, 1H), 6.64 (m, 1H), 6.78 (s, 1H), 6.93 (apparent t, *J* =7.8, 1H).

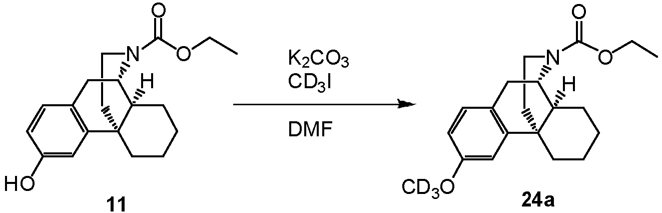

*(+)-17-ethylcarbamate-3-d₃-methoxy-(9α,13α,14α)-morphinan (**24a;** R³*= *-OCD₃).* The compound **11** (1.48 g, 4.7 mmol) was dissolved in DMF (20 mL) in a reaction vessel fit with a stir bar. To this solution was added K₂CO₃ (2.97 g, 21.5 mmol). The mixture was stirred under an atmosphere of N₂ at RT for 10 min before the addition of CD₃I (1.02 g, 7.0 mmol). The resulting reaction mixture was stirred overnight at RT at which time tlc (20% ethylacetate/hexane) showed complete reaction. The mixture was diluted with H₂O then was extracted with ethyl ether (3 x 30 mL). The combined organic layers were dried over Na₂SO₄, filtered, and the filtrate concentrated on a rotary evaporator to a clear yellow oil. Purification via automated flash column chromatography (0-20% ethylacetate/hexane) and concentration of pure fractions on a rotary evaporator afforded 793 mg of product.

**¹H-NMR** (300 MHz, CDCl₃): δ 1.01-1.11 (m, 1H), 1.22-1.39 (m, 7H), 1.45-1.59 (m, 3H), 1.62-1.70 (m, 2H), 2.34-2.37 (m, 1H), 2.54-2.69 (m, 2H), 3.04-3.12 (m, 1H), 3.84 (ddd, *J₁*=43.2, *J₂*=13.8, *J₃*=4.8, 1H), 4.09-4.17 (m, 2H), 4.31 (dt, *J₁*=56.4, *J₂*=3.4, 1H), 6.71 (dd, *J₁*=8.4, *J₂*=2.5, 1H), 6.82 (d, *J*=2.7, 1H), 7.00 (apparent t, *J*=8.2, 1H).

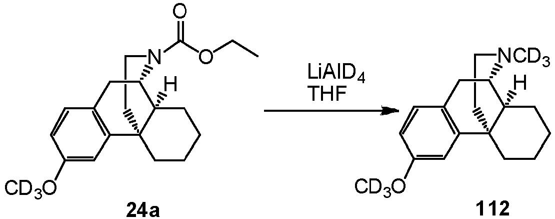

*(+)-3-d₃-methoxy-17-d₃-methyl-(9α,13α,14α)-morphinan (Compound **112).*** To a reaction vessel fit with a stir bar, was added THF (5 mL) and LAD (100 mg, 2.4 mmol). The slurry was cooled to 0 °C followed by the addition of a solution of product **24a** (R³= -OCD₃, 397 mg, 1.2 mmol) in THF (5 mL). The reaction mixture was stirred under an atmosphere of N₂ for 2 h at which time tlc (20% ethylacetate/hexane) showed the reaction to be complete. The mixture was then quenched by the addition of magnesium sulfate heptahydrate until cessation of gas evolution. Ethyl ether (25 mL) was added to the flask, the slurry was filtered, and the organic filtrate was concentrated on a rotary evaporator to an oil. The crude product was purified via automated flash column chromatography (CH₃OH with 1% NH₄OH / CHCl₃, 0-10%), concentrated on a rotary evaporator, then dissolved in a saturated solution of HBr in dioxane. The mixture was stirred for 10 min, was concentrated on a rotary evaporator, then dried under vacuum for 3 d to yield 204 mg of Compound **112.**

**¹H-NMR** (300 MHz, CDCl₃): δ 1.08 (ddd, *J₁*=25.1, *J₂*=12.6, *J₃=*3.3, 1H), 1.22-1.32 (m, 1H), 1.35-1.48 (m, 4H), 1.60 (dd, *J₁*=39.0, *J₂*=12.6, 2H), 2.02 (dt, *J₁*=13.2, *J₂*=4.0, 1H), 2.17 (d, *J*=11.9, 1H), 2.34 (t, *J*=13.5, 2H), 2.75-2.80 (m, 1H), 2.88 (dd, *J₁*=18.8, *J*₂=5.3, 1H), 3.01 (d, *J*=18.5, 1H), 3.15 (s, 1H), 6.73 (d, *J*=8.6, 1H), 6.81 (s, 1H), 7.05 (d, *J*=8.6, 1H). **HPLC** (method: 150 mm C18-RP column - gradient method 5-95% ACN; Wavelength: 254 nm): retention time: 6.74 min. **MS** (M+H⁺): 278.4.

### Example 6 (reference). Synthesis of (+)-3-methoxy-d₃-17-methyl-(9α,13α,14α)-morphinan (111).

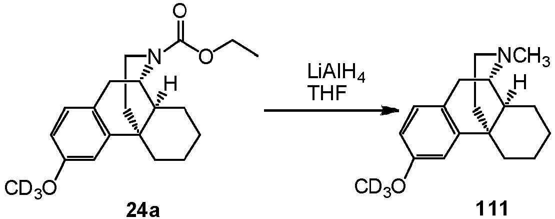

*(+)-3-d₃-methoxy-17-methyl-(9α,13α,14α)-morphinan (Compound **111).*** To a reaction vessel fit with a stir bar, was added THF (5 mL) and LAH (91 mg, 2.4 mmol). The slurry was cooled to 0 °C followed by the addition of product **24a** (R³= -OCD₃, 397 mg, 1.2 mmol) dissolved in THF (5 mL). The reaction mixture was stirred under an atmosphere of N₂ for 2 h at which time tlc (20% ethylacetate/hexane) showed the reaction to be complete. The mixture was then quenched by the addition of magnesium sulfate heptahydrate until cessation of gas evolution. Ethyl ether (25 mL) was added to the flask, the slurry was filtered, and the organic filtrate was concentrated on a rotary evaporator to an oil. The crude product was purified via automated flash column chromatography (CH₃OH with 1% NH₄OH / CHCl₃, 0-10%), concentrated on a rotary evaporator, then dissolved in a saturated solution of HBr in dioxane. The mixture was stirred for 10 min, was concentrated on a rotary evaporator, then dried under vacuum for 3 d to yield 200 mg of Compound **111.**

**¹H-NMR** (300 MHz, CDCl₃): δ 1.07-1.16 (m, 1H), 1.22-1.32 (m, 1H), 1.34-1.46 (m, 4H), 1.59 (dd, *J₁*=41.0, *J₂*=12.6, 2H), 1.94 (t, *J*=12.6, 1H), 2.06 (d, *J*=12.9, 1H), 2.26 (t, *J*=12.6, 1H), 2.36 (d, *J*=13.2, 1H), 2.53 (s, 3H), 2.67 (d, *J*=12.2, 1H), 2.78 (dd, *J₁*=18.8, *J₂*=5.0, 1H), 3.06 (d, *J*=19.2, 2H), 6.72 (d, *J*=8.3, 1H), 6.81 (s, 1H), 7.05 (d, *J* =8.6, 1H). **HPLC** (method: 150 mm C18-RP column - gradient method 5-95% ACN; Wavelength: 254 nm): retention time: 6.86 min. **MS** (M+H⁺): 275.2.

### Example 7 (reference). Synthesis of (+)-3-methoxy -17-(methyl-d₃)-(9α,13α,14α)-morphinan (119).

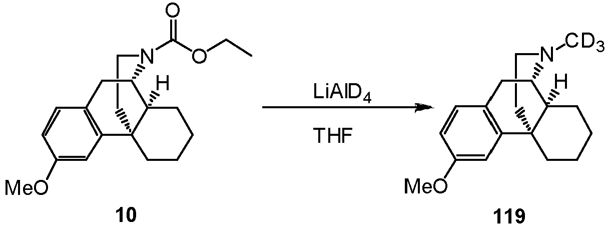

*(+)-3-methoxy-17-d₃-methyl-(9α,13α,14α)-morphinan (Compound **119).*** To a reaction vessel fit with a stir bar, was added THF (2 mL) and LAD (99 mg, 2.4 mmol). The slurry was cooled to 0 °C followed by the gradual addition of carbamate **10** (195 mg, 6.0 mmol) dissolved in THF (3 mL). The reaction mixture was stirred under an atmosphere of N₂ for 10 min at which time tlc (20% ethylacetate/hexane) showed the reaction to be complete. The mixture was then quenched by the addition of magnesium sulfate heptahydrate until cessation of gas evolution. The resulting solid was washed with ethyl ether, filtered, and the organic filtrate was concentrated on a rotary evaporator to an oil. The crude product was purified via automated flash column chromatography (CH₃OH with 1% NH₄OH / CHCl₃, 90%), concentrated on a rotary evaporator, and then dissolved in a saturated solution of HBr in dioxane. The mixture was stirred for 10 min, and then concentrated on a rotary evaporator to yield 74 mg of product.

**¹H-NMR** (300 MHz, CDCl₃): δ 0.96 (ddd, *J₁*=25.4, *J₂*=12.7, *J₃*=3.9, 1H), 1.08-1.18 (m, 1H), 1.24-1.36 (m, 2H), 1.43-1.52 (m, 3H), 1.62 (d, *J*=12.7, 1H), 1.78 (td, *J₁*=13.7, *J₂*=4.4, 1H), 1.96 (d, *J*=12.2, 1H), 2.41-2.47 (m, 2H), 2.97 (dd, *J₁*=19.5, *J₂*=5.9, 1H), 3.10-3.18 (m, 2H), 3.60-3.63 (m, 1H), 3.73 (s, 3H), 6.81-6.84 (m, 2H), 7.13 (d, *J*=9.3, 1H), 9.60 (bs, 1H). **HPLC** (method: 150 mm C18-RP column - gradient method 5-95% ACN; Wavelength: 280 nm): retention time: 6.91 min. **MS** (M+H⁺): 275.7.

**Example 8 (reference).** Evaluation of Metabolic Stability in CYP2D6 SUPERSOMES^{™}. Human CYP2D6 SUPERSOMES^{™} were purchased from GenTest (Woburn, MA, USA). 7.5 mM stock solutions of test compounds (Compounds 100, 102, 104, 106, dextromethorphan, a deuterated analog of dextromethorphan wherein each methyl group was replaced with CD₃ ("d₆-dextromethorphan", chemical name (+)-3-d₃-methoxy-17-d₃-methyl-(9α,13α,14α)-morphinan, also referred to as Compound 101 in U.S. Ser. No. 12/112,936, and as "Test Compound" in Figure 1 and Table 2 below), the ethyl ether analog of dextromethorphan ("dextroethorphan") or the isopropyl ether analog of dextromethorphan ("dextroisoproporphan")) were prepared in DMSO. The 7.5 mM stock solutions were diluted to 50 µM in acetonitrile (ACN). The 1000 pmol/mL CYP2D6 SUPERSOMES^{™} were diluted to 62.5 pmol/mL in 0.1 M potassium phosphate buffer, pH 7.4, containing 3 mM MgCl₂. The diluted SUPERSOMES^{™} were added to wells of a 96-well deep-well polypropylene plate in triplicate. 10 µL of the 50 µM test compound was added to the SUPERSOMES^{™} and the mixture was pre-warmed for 10 minutes. Reactions were initiated by addition of pre-warmed NADPH solution. The final reaction volume was 0.5 mL and contained 50 pmol/mL CYP2D6 SUPERSOMES^{™}, 1 µM test compound, and 2 mM NADPH in 0.1 M potassium phosphate buffer, pH 7.4, and 3 mM MgCl₂. The reaction mixtures were incubated at 37°C and 50 µL aliquots were removed at 0, 5, 10, 20, and 30 minutes and added to shallow-well 96-well plates which contained 50 µL of ice-cold ACN with internal standard to stop the reactions. The plates were stored at 4°C for 20 minutes after which 100 µL of water was added to the wells of the plate before centrifugation to pellet precipitated proteins. Supernatants were transferred to another 96-well plate and analyzed for amounts of parent remaining by LC-MS/MS using an Applied Bio-systems API 4000 mass spectrometer.

The *in vitro* half-life (t_{1/2}) for each of the test compounds was calculated from the slopes of the linear regression of % parent remaining (ln) vs incubation time relationship: in vitro t ½ = 0.693/k, where k = -[slope of linear regression of % parent remaining(ln) vs incubation time]. Data analysis was performed using Microsoft Excel Software..

Figure 1 and Table 2, below, show the results of the SUPERSOMES^{™} experiment. Note that in Figure 1, the curves for Compounds 100 and 104 overlap one another. "Test Compound" in Figure 1 and Table 2 refers to deuterated dextromethorphan ("d6-dextromethorphan", (+)-3-d3-methoxy-17-d3-methyl-(9α,13α,14α)-morphinan, which is also referred to as Compound 101 in U.S. Ser. No. 12/112,936).

**Table 2. Calculated Half-life in SUPERSOMES^{™},**

| **Compound** | **t_{1/2} ± SD (min)** |
|---|---|
| Dextromethorphan | 1.7 ± 0.3 |
| Test Compound | 5.6 ± 1.5 |
| Dextroethorphan | 10.3 ± 2.1 |
| Dextroisoproporphan | 21.7 ± 1.6 |
| Compound 106 | 36.0 ± 2.8 |
| Compound 102 | 39.0 ± 1.9 |
| Compound 104 | 49.1 ± 4.1 |
| Compound 100 | 51.3 ± 3.7 |

Each of the deuterated compounds tested demonstrated a longer half-life when incubated with CYP2D6 SUPERSOMES^{™} than any of the corresponding undeuterated test compounds or a deuterated version of dextromethorphan (Test Compound). Thus, in this assay, the compounds of this disclosure were more resistant to metabolism than dextromethorphan or deuterated dextromethorphan (Test Compound).

## Claims

1. A combination of:
(a) a therapeutically effective amount of a selective serotonin reuptake inhibitor; and
(b) a therapeutically effective amount of a compound of Formula **II:** 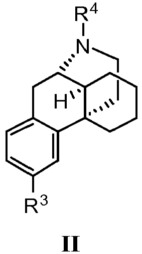 or a pharmaceutically acceptable salt thereof, wherein:
R³ is selected from -OCH₂D, -OCHD₂, and -OCD₃;
R⁴ is selected from -CH₃, -CH₂D, -CHD₂, and -CD₃;
provided that when R³ is -OCD₃, then R⁴ is not -CH₃;
for use in treating chronic or intractable pain.

2. The combination for use of claim 1, wherein R⁴ is -CH₃, -CHD₂, or -CD₃.

3. The combination for use of claim 1, wherein R³ is -OCD₃.

4. The combination for use of claim 1, wherein the selective serotonin reuptake inhibitor is an inhibitor of a cytochrome p450 2D6 enzyme.

5. The combination for use of any one of claims 1 to 4, wherein the selective serotonin reuptake inhibitor is selected from the group consisting of fluoxetine, norfluoxetine, citalopram, dapoxetine, escitalopram, fluvoxamine, paroxetine, and sertraline, or pharmaceutically acceptable salts thereof.

6. The combination for use of claim 5, wherein the selective serotonin reuptake inhibitor is selected from the group consisting of citalopram, norfluoxetine, dapoxetine, escitalopram, fluvoxamine, paroxetine, and sertraline, or pharmaceutically acceptable salts thereof.

7. The combination for use of claim 6, wherein the selective serotonin reuptake inhibitor is paroxetine, or a pharmaceutically acceptable salt thereof.

8. The combination for use of claim 1, wherein the selective serotonin reuptake inhibitor is selected from the group consisting of citalopram, fluvoxamine, norfluoxetine, fluoxetine, paroxetine and sertraline, and the compound of formula II is selected from the group consisting of: 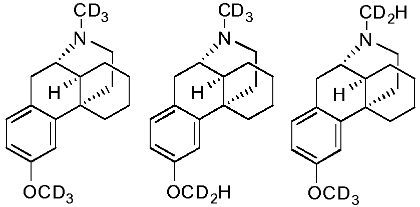 or pharmaceutically acceptable salts thereof.

9. The combination for use of claim 8, wherein the selective serotonin reuptake inhibitor is selected from the group consisting of citalopram, fluvoxamine, paroxetine and sertraline, or a pharmaceutically acceptable salt thereof.

10. The combination for use of claim 8 or claim 9, wherein the selective serotonin reuptake inhibitor is paroxetine, or a pharmaceutically acceptable salt thereof.

## Patentansprüche

1. Kombination von:
(a) einer therapeutisch wirksamen Menge eines selektiven Serotoninwiederaufnahmehemmers; und
(b) einer therapeutisch wirksamen Menge einer Verbindung der Formel II: 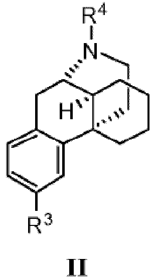 oder eines pharmazeutisch verträglichen Salzes davon, wobei:
R³ aus -OCH₂D, -OCHD₂ und -OCD₃ ausgewählt ist;
R⁴ aus -CH₃, -CH₂D, -CHD₂ und -CD₃ ausgewählt ist;
mit der Maßgabe, dass, wenn R³ -OCD₃ ist, R⁴ nicht -CH₃ ist;
zur Verwendung bei der Behandlung von chronischen oder hartnäckigen Schmerzen.

2. Kombination zur Verwendung nach Anspruch 1, wobei R⁴ -CH₃, -CHD₂ oder -CD₃ ist.

3. Kombination zur Verwendung nach Anspruch 1, wobei R³ -OCD₃ ist.

4. Kombination zur Verwendung nach Anspruch 1, wobei der selektive Serotoninwiederaufnahmehemmer ein Inhibitor eines Cytochrom-p450-2D6-Enzyms ist.

5. Kombination zur Verwendung nach einem der Ansprüche 1 bis 4, wobei der selektive Serotoninwiederaufnahmehemmer ausgewählt ist aus der Gruppe bestehend aus Fluoxetin, Norfluoxetin, Citalopram, Dapoxetin, Escitalopram, Fluvoxamin, Paroxetin und Sertralin oder pharmazeutisch verträglichen Salzen davon.

6. Kombination zur Verwendung nach Anspruch 5, wobei der selektive Serotoninwiederaufnahmehemmer ausgewählt ist aus der Gruppe bestehend aus Citalopram, Norfluoxetin, Dapoxetin, Escitalopram, Fluvoxamin, Paroxetin und Sertralin oder pharmazeutisch verträglichen Salzen davon.

7. Kombination zur Verwendung nach Anspruch 6, wobei der selektive Serotoninwiederaufnahmehemmer Paroxetin oder ein pharmazeutisch verträgliches Salz davon ist.

8. Kombination zur Verwendung nach Anspruch 1, wobei der selektive Serotoninwiederaufnahmehemmer ausgewählt ist aus der Gruppe bestehend aus Citalopram, Fluvoxamin, Norfluoxetin, Fluoxetin, Paroxetin und Sertralin, und die Verbindung der Formel II ausgewählt ist aus der Gruppe bestehend aus: 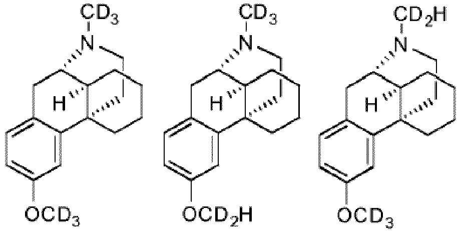 oder pharmazeutisch verträglichen Salzen davon.

9. Kombination zur Verwendung nach Anspruch 8, wobei der selektive Serotoninwiederaufnahmehemmer ausgewählt ist aus der Gruppe bestehend aus Citalopram, Fluvoxamin, Paroxetin und Sertralin oder einem pharmazeutisch verträglichen Salz davon.

10. Kombination zur Verwendung nach Anspruch 8 oder Anspruch 9, wobei der selektive Serotoninwiederaufnahmehemmer Paroxetin oder ein pharmazeutisch verträgliches Salz davon ist.

## Revendications

1. Combinaison de :
(a) une quantité thérapeutiquement efficace d'un inhibiteur sélectif de la recapture de la sérotonine ; et
(b) une quantité thérapeutiquement efficace d'un composé de Formule II : 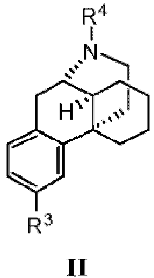 ou un sel pharmaceutiquement acceptable de celui-ci, dans lequel :
R³ est choisi parmi -OCH₂D, -OCHD₂ et -OCD₃ ;
R⁴ est choisi parmi -CH₃, -CH₂D, -CHD₂ et -CD₃;
à condition que lorsque R³ est -OCD₃, alors R⁴ n'est pas -CH₃ ;
pour utilisation dans le traitement de la douleur chronique ou réfractaire.

2. Combinaison pour utilisation selon la revendication 1, dans laquelle R⁴ est -CH₃, -CHD₂ ou -CD₃.

3. Combinaison pour utilisation selon la revendication 1, dans laquelle R³ est -OCD₃.

4. Combinaison pour utilisation selon la revendication 1, dans laquelle ledit inhibiteur sélectif de la recapture de la sérotonine est un inhibiteur d'une enzyme cytochrome p450 2D6.

5. Combinaison pour utilisation selon l'une quelconque des revendications 1 à 4, dans laquelle ledit inhibiteur sélectif de la recapture de la sérotonine est choisi dans le groupe constitué de fluoxétine, norfluoxétine, citalopram, dapoxétine, escitalopram, fluvoxamine, paroxétine et sertraline ou de sels pharmaceutiquement acceptables de ceux-ci.

6. Combinaison pour utilisation selon la revendication 5, dans laquelle ledit inhibiteur sélectif de la recapture de la sérotonine est choisi dans le groupe constitué de citalopram, norfluoxétine, dapoxétine, escitalopram, fluvoxamine, paroxétine et sertraline ou de sels pharmaceutiquement acceptables de ceux-ci.

7. Combinaison pour utilisation selon la revendication 6, dans laquelle ledit inhibiteur sélectif de la recapture de la sérotonine est de la paroxétine ou un sel pharmaceutiquement acceptable de celle-ci.

8. Combinaison pour utilisation selon la revendication 1, dans laquelle ledit inhibiteur sélectif de la recapture de la sérotonine est choisi dans le groupe constitué de citalopram, fluvoxamine, norfluoxétine, fluoxétine, paroxétine et sertraline, et le composé de formule II est choisi dans le groupe constitué de : 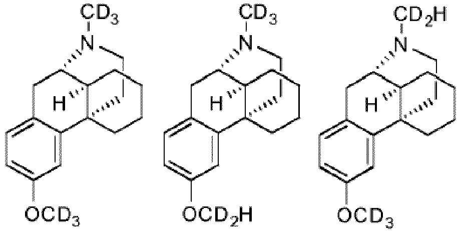 ou de sels pharmaceutiquement acceptables de ceux-ci.

9. Combinaison pour utilisation selon la revendication 8, dans laquelle ledit inhibiteur sélectif de la recapture de la sérotonine est choisi dans le groupe constitué de citalopram, fluvoxamine, paroxétine et sertraline ou un sel pharmaceutiquement acceptable de ceux-ci.

10. Combinaison pour utilisation selon la revendication 8 ou la revendication 9, dans laquelle ledit inhibiteur sélectif de la recapture de la sérotonine est de la paroxétine ou un sel pharmaceutiquement acceptable de celle-ci.
